(19) Europäisches Patentamt
European Patent Office
Office européen des brevets

(11) Veröffentlichungsnummer : **0 024 016**
**B1**

(12) **EUROPÄISCHE PATENTSCHRIFT**

(45) Veröffentlichungstag der Patentschrift : **24.02.82**

(21) Anmeldenummer : **80104524.6**

(22) Anmeldetag : **31.07.80**

(51) Int. Cl.³ : **C 07 C103/76**, C 07 C149/42, A 01 N 37/30// C07D317/62, C07D319/08, C07D319/20

(54) **Tetrachlorphthalamidsäuren, Verfahren zu ihrer Herstellung und ihre bakterizide Verwendung.**

(30) Priorität : **11.08.79 DE 2932689**

(43) Veröffentlichungstag der Anmeldung : **18.02.81 (Patentblatt 81/07)**

(45) Bekanntmachung des Hinweises auf die Patenterteilung : **24.02.82 Patentblatt 82/08**

(84) Benannte Vertragsstaaten : **BE CH DE FR GB IT LI NL**

(56) Entgegenhaltungen :
**EP - A - 0 011 179**
**DE - A - 1 768 244**
**DE - A - 2 040 578**
**GB - A - 1 355 849**

(73) Patentinhaber : **BAYER AG**
**Zentralbereich Patente, Marken und Lizenzen**
**D-5090 Leverkusen 1, Bayerwerk (DE)**

(72) Erfinder : **Kühle, Engelbert, Dr.**
**Von Bodelschwingh-Strasse 42**
**D-5060 Bergisch-Gladbach 2 (DE)**
Erfinder : **Kraus, Peter, Dr.**
**Duesseldorfer Strasse 43**
**D-5000 Koeln 80 (DE)**
Erfinder : **Marhold, Albrecht, Dr.**
**Carl-Duisberg-Strasse 329**
**D-5090 Leverkusen 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 024 016 B1

Imprimerie Jouve, 18, rue St-Denis, 75001 Paris, France

Tetrachlorphthalamidsäuren, Verfahren zu ihrer Herstellung und ihre bakterizide Verwendung

Die vorliegende Erfindung betrifft neue Tetrachlorphthalamidsäuren, ein Verfahren zu ihrer Herstellung, sowie ihre Verwendung als Bakterizide im Pflanzenschutz.

Es ist bereits bekannt geworden, daß Tetrachlorphthalamidsäuren eine pflanzenbakterizide Wirksamkeit besitzen. So zeigt die N-(2,3-Dichlorphenyl)-tetrachlorphthalamidsäure eine bakterizide Wirkung gegen Xanthomonas oryzae im Reisanbau (vgl. z.B. GB-PS 1 355 849). Die Wirksamkeit ist jedoch bei niedrigen Anwendungskonzentrationen nicht immer befriedigend.

Es wurden neue Tetrachlorphthalamidsäuren der allgemeinen Formel

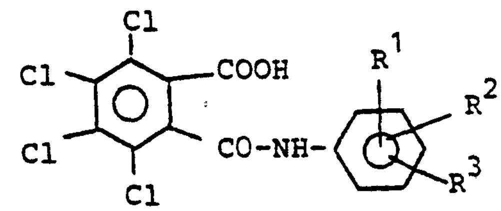

(I)

in welcher

$R^1$ für Trihalogenmethyl-, Trihalogenmethoxi- oder Trihalogenmethylmercapto-Gruppen steht,

$R^2$ für Wasserstoff, Halogen, Niederalkyl- oder Niederalkoxi-Gruppen steht, oder zusammen mit $R^1$ in o-Stellung $-O-CF_2-O-CF_2-$, $-O-CF_2-O-$ und $-O-CF_2-CFX-O-$ bedeuten, wobei X für Wasserstoff, Chlor oder Fluor steht, und

$R^3$ für Wasserstoff, Halogen, Niederalkyl-, Niederalkoxi-, Niederalkylmercapto- oder Aroxi-Gruppen steht, wobei letzere gegebenenfalls durch Halogen, Methyl- und/oder Methoxygruppen substituiert sein können,

gefunden.

Weiterhin wurde gefunden, daß man die neuen Tetrachlorphthalamidsäuren der Formel (I) erhält, wenn man Tetrachlorphthalsäureanhydrid der Formel

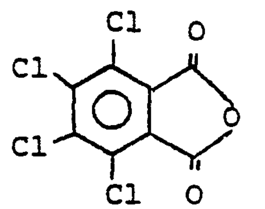

(II)

mit einem Amin der Allgemeinen Formel

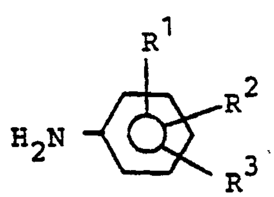

(III)

in welcher

$R^1$, $R^2$ und $R^3$ die oben angegebene Bedeutung besitzen,

in Gegenwart eines Verdünnungsmittels umsetzt.

Die neuen Tetrachlorphthalamidsäuren weisen starke bakterizide Wirkungen auf. Dabei ist es überraschend, daß die erfindungsgemäßen Verbindungen eine bessere Wirkung gegen pflanzenschädigende Bakterien aufweisen als die aus dem Stande der Technik bekannten Produkte. Die neuen Verbindungen stellen somit eine Bereicherung der Technik dar.

Von den Tetrachlorphthalamidsäuren der Formel (I) sind diejenigen bevorzugt, in denen $R^1$ für eine Trifluormethyl-, Trifluormethoxi-, Trifluormethylmercapto-, Difluorchlormethyl-, Difluorchlormethoxi- oder Difluorchlormethylmercapto-Gruppe steht, $R^2$ für Wasserstoff, Chlor, Fluor, eine Methyl- oder Methoxi-Gruppe steht, oder aber $R^1$ und $R^2$ zusammen in ortho-Stellung für die Gruppen $-CF_2-O-CF_2-O-$, $-O-CF_2-O-$, $-O-CF_2-CF_2-O-$, $-O-CF_2-CFCl-O-$ oder $-O-CF_2-CHF-O-$ stehen, und $R^3$ für Wasserstoff, Chlor, eine Methyl-, Methoxi-, Methylmercapto- oder Phenoxi-Gruppe steht, wobei letztere durch Halogen, Methyl- und/oder Methoxi-Gruppen substituiert sein kann.

Verwendet man zur Herstellung der erfindungsgemäßen Verbindungen beispielsweise Tetrachlorphthalsäureanhydrid und 4-Trifluormethylanilin als Ausgangsstoffe, so kann der Reaktionsablauf durch

das folgende Formelschema wiedergegeben werden :

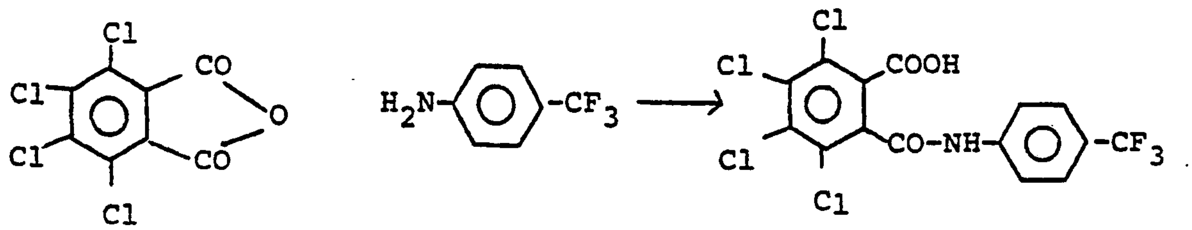

Selbstverständlich können die freien Säuren der allgemeinen Formel I in geeignete Salze, wie z.B. Alkali-, Erdalkali- oder Ammonium-Salze überführt werden.

Das als Ausgangsstoff zu verwendende, allgemein bekannte Tetrachlorphthalsäureanhydrid ist durch die Formel (II) bezeichnet.

Die weiterhin als Ausgangsstoffe zu verwendenden Amine sind durch die allgemeine Formel (III) definiert. In dieser Formel haben $R^1$, $R^2$ und $R^3$ die für die allgemeine Formel (I) oben angegebenen vorzugsweisen Bedeutungen.

Die Amine der Formel (III) und deren Herstellung sind bekannt oder — soweit sie noch nicht bekannt sind — nach literaturbekannten Verfahren herstellbar (vgl. z.B. J. Org. Chem. *25* (1960), 965 und *29* (1964), 1 ; Z. Obsc. Chim. (Engl. Transl.) *31* (1961), 578 ; Angew. Chem. *89* (1977), 797 ; vgl. auch die Angaben bei den Herstellungsbeispielen). Als Beispiele für Verbindungen der Formel (III) seien genannt :

2-, 3- oder 4-Trifluormethyl-anilin, 3-Chlor-4-trifluormethyl-anilin 2-Trifluormethyl-4-methylmercapto-anilin, 3-Trifluormethyl-4-chlorphenoxi-anilin, 2-, 3- oder 4-Trifluormethoxi-anilin, 3-Chlor-4-trifluormethoxi-anilin, 2-, 3- oder 4-Trifluormethylmercapto-anilin, 3-Chlor-4-trifluormethylmercapto-anilin, 3-Chlor-4-difluorchlormethylmercapto-anilin, 5-Amino-2,2-difluorbenzdioxol, 6-Amino-tetrafluorbenzdioxen-1,3,6-Amino-trifluorbenzdioxen-1,4.

Als Verdünnungsmittel kommen alle inerten Lösungsmittel in Frage. Hierzu gehören Ether wie Tetrahydrofuran und Dioxan, Kohlenwasserstoffe wie Toluol, Chlorkohlenwasserstoffe wie Chloroform oder Ketone wie Aceton.

Die Reaktionstemperaturen können in einem größeren Bereich variiert werden, im allgemeinen arbeitet man zwischen 20 und 120 °C, vorzugsweise bei 50 bis 100 °C.

Bei der Durchführung des Verfahrens arbeitet man im allgemeinen in molaren Mengen, jedoch schadet ein Aminüberschuß bis etwa 10 % nicht.

Die Aufarbeitung erfolgt in üblicher Weise. Die Reaktionsprodukte sind kristalline Verbindungen, die durch Filtration isoliert werden können.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Die erfindungsgemäßen Verbindungen sind besonders gut wirksam gegen bakterizide Pflanzenkrankheiten.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Insbesondere sind die erfindungsgemäßen Verbindungen wirksam gegen Bakterien der Gattung Xanthomonas, z.B. gegen Xanthomonas oryzae an Reis. Bei der Bekämpfung von bakteriellen Krankheiten ist von Vorteil, daß die erfindungsgemäßen Verbindungen systemische Eigenschaften aufweisen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

## Beispiel A

### Agarplatten-Test

Verwendeter Nährboden :

15 Gewichtsteile : AgarAgar
10 Gewichtsteile : Saccharose
8 Gewichtsteile : Caseinhydrolysat
4 Gewichtsteile : Hefeextrakt
2 Gewichtsteile : Dikaliumhydrogenphosphat
0,3 Gewichtsteile : Magnesiumphosphat

werden im 1 000 Gewichtsteilen destilliertem Wasser gelöst und 30 Minuten bei 121 °C im geschlossenen Gefäß sterilisiert.

Lösungsmittel : 40 Gewichtsteile Dimethylformamid Mengenverhältnis von Lösungsmittel zu Nährboden : 2: 100.

Zur Herstellung einer zweckmäßigen Wirkstoffzubereitung vermischt man 1 Gewichtsteil Wirkstoff mit der angegebenen Menge Lösungsmittel.

Das Konzentrat wird im genannten Mengenverhältnis mit dem flüssigen Nährboden gründlich vermischt und in Petrischalen gegossen.

Ist der Nährboden erkaltet und fest, werden die Platten mit folgenden Mikroorganismen beimpft und bei ca. 21 °C inkubiert :

Erwinia mangiferae
Xanthomonas oryzae

Xanthomonas pelargonii
Agrobacterium tumefaciens
Corynebacterium michiganense

Die Auswertung erfolgt je nach Wachstumsgeschwindigkeit der Mikroorganismen nach 2 bis 8 Tagen, wobei die Wachstumshemmung im Vergleich zur unbehandelten Kontrolle als Maß für die Wirkung der Präparate herangezogen wird.

Die Bonitierung des Wachstums geschieht mit folgenden Kennzahlen :

1 kein Wachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Die nachfolgende Tabelle zeigt die Versuchsergebnisse auf :

Tabelle A
Agarplatten-Test

| Wirkstoffe | Wirkstoffkonz. [ppm] | Bakterienwachstum in Wertzahlen: Erwinia mangiferae | Xanthomonas oryzae | Xanthomonas pelargonii | Agrobacterium tumefaciens | Corynebacterium michiganense |
|---|---|---|---|---|---|---|
| 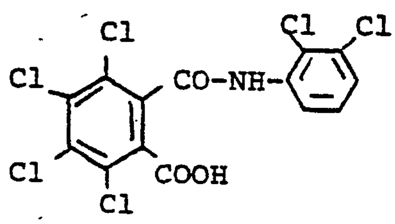 (bekannt) | 500 | — | — | 9 | 9 | 9 |
| | 250 | 5 | 5 | — | — | — |
| 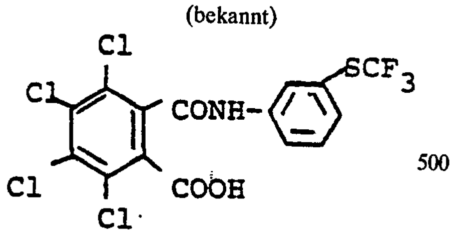 (9) | 500 | — | — | — | 3 | 1 |
| 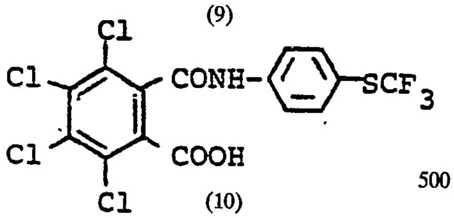 (10) | 500 | — | — | — | 2 | 1 |
| 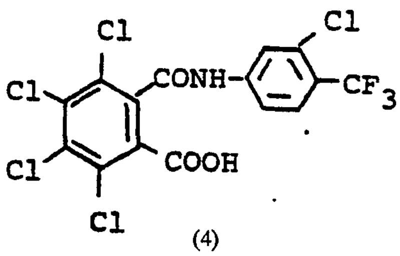 (4) | 500 | — | — | 2 | — | 1 |

Tabelle A (Fortsetzung)
Agarplatten-Test

| Wirkstoffe | Wirkstoffkonz. [ppm] | Bakterienwachstum in Wertzahlen: Erwinia mangiferae | Xanthomonas oryzae | Xanthomonas pelargonii | Agrobacterium tumefaciens | Corynebacterium michiganense |
|---|---|---|---|---|---|---|
| 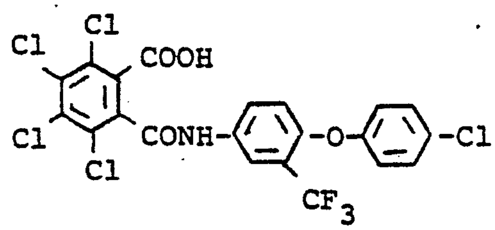 (13) | 500 | — | — | 2 | — | 1 |
| | 250 | 1 | 1 | — | — | — |
| 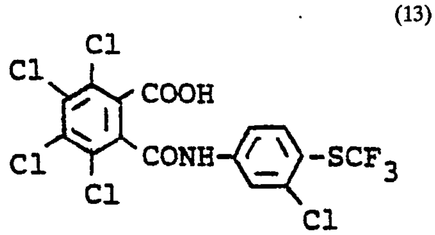 (11) | 500 | — | — | 1 | 1 | 1 |
| | 250 | 1 | 1 | — | — | — |
| 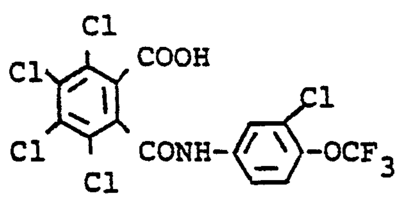 (8) | 500 | — | — | — | 3 | 1 |

Beispiel B

Xanthomonas oryzae-Test/Bakteriose/Reis/protektiv

Lösungsmittel : 25 Gewichtsteile : Aceton
Emulgator : 0,75 Gewichtsteile : Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach dem Antrocknen des Spritzbelages werden die Pflanzen mit einer wäßrigen Suspension von Xanthomonas oryzae durch Stichverletzung der Blätter inokuliert. Nach einer 48-stündigen Inkubation bei 100 % rel. Luftfeuchtigkeit verbleiben die Pflanzen bis zur Auswertung in einem Gewächshaus bei 24 bis 26 °C und 70 bis 80 % rel. Luftfeuchtigkeit.

10 Tage nach der Inokulation wird der Befall bei allen inokulierten Pflanzen in Prozent des Befalls der unbehandelten Kontrollpflanzen ausgedrückt. 0 % bedeutet keinen Befall, 100 % einen Befall entsprechend dem Befall der unbehandelten Kontrollpflanzen.

Die nachfolgende Tabelle zeigt die Versuchsergebnisse auf.

Tabelle B
Xanthomonas oryzae-Test / Bakteriose / Reis / protektiv

| Wirkstoffe | Wirkstoff-konzentration in % | Krankheitsbefall in % der unbehandelten Kontrolle |
|---|---|---|
| 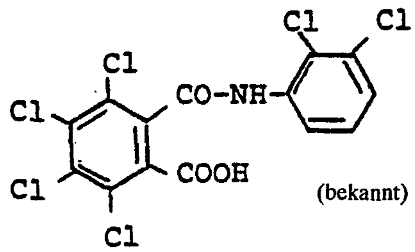 (bekannt) | 0,05 | 50 |
| 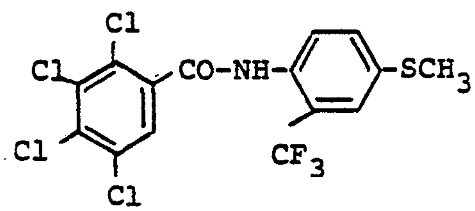 (12) | 0,05 | 25 |
| 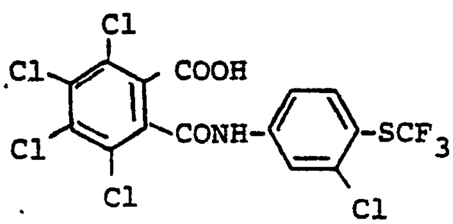 (11) | 0,05 | 12,5 |
| 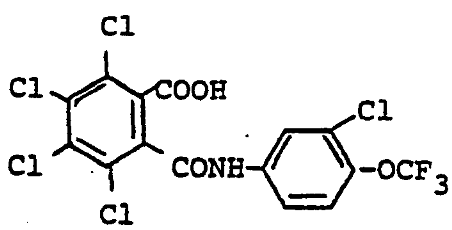 (8) | 0,05 | 12,5 |
| 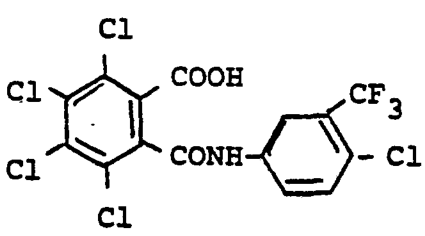 (5) | 0,05 | 25 |

## Herstellungsbeispiele

### Beispiel 1

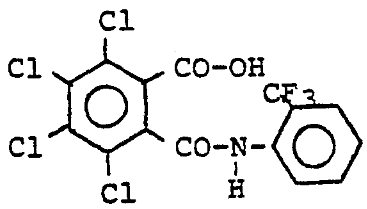

20 g (0,07 Mol) Tetrachlorphthalsäureanhydrid (Fp 255-257 °C) werden bei 80 °C in 100 ml Dioxan gelöst und bei dieser Temperatur mit 14,4 g (0,07 Mol) 2-Trifluormethylanilin in 30 ml Dioxan tropfenweise versetzt. Man erhitzt 1 Stunde lang zum Sieden und saugt das Reaktionsprodukt in der Kälte ab. Man erhält 16 g N-(2-Trifluormethyl-phenyl)-tetrachlorphthalamidsäure vom Fp 205 °C (unter Zersetzung), das sind 52 % der Theorie.

In entsprechender Weise lassen sich die folgenden Verbindungen der allgemeinen Formel

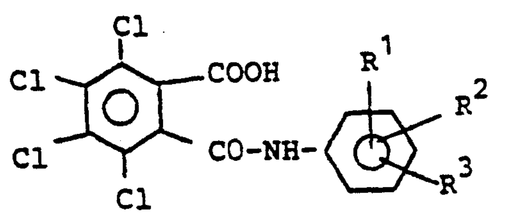

(I)

herstellen :

| Beispiel Nr. | $R^1$ | $R^2$ | $R^3$ | Fp (°C) |
|---|---|---|---|---|
| 2 | 3—$CF_3$ | H | H | 190 (Zers.) |
| 3 | 4—$CF_3$ | H | H | 210 (Zers.) |
| 4 | 4—$CF_3$ | 3—Cl | H | 160 (Zers.) |
| 5 | 3—$CF_3$ | 4—Cl | H | 275-276 |
| 6 | 3—$OCF_3$ | H | H | 155 (Zers.) |
| 7 | 4—$OCF_3$ | H | H | 210 (Zers.) |
| 8 | 4—$OCF_3$ | 3—Cl | H | 247-248 |
| 9 | 3—$SCF_3$ | H | H | 199-202 |
| 10 | 4—$SCF_3$ | H | H | 218 (Zers.) |
| 11 | 4—$SCF_3$ | 3—Cl | H | 211-212 |
| 12 | 2—$CF_3$ | H | 4—S—$CH_3$ | 210 (Zers.) |
| 13 | 3—$CF_3$ | H | 4—O—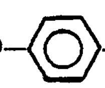—Cl | 160 (Zers.) |
| 14 | 3,4—$CF_2$—O—$CF_2$—O— | | H | 262-263 |
| 15 | 3,4—O—CFCl—$CF_2$—O— | | H | 238-242 |
| 16 | 3,4—O—CHF—$CF_2$—O— | | H | 259-262 |
| 17 | 3,4—O—$CF_2$—O— | | H | 261-263 |
| 18 | 3,4—O—CHF—$CF_2$—O— | | 6—Cl | 278 |
| 19 | 3,4—O—$CF_2$—CHF—O— | | H | 236 |
| 20 | $CF_2ClO$ | H | H | 234 |

## Vorprodukte

Bei Darstellung der als Ausgangsstoffe benötigten Amine der allgemeinen Formel (III) kann beispielsweise wie folgt verfahren werden :

Beispiel V 1

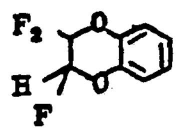

In 600 ml Tetramethylensulfon werden 220 g Brenzkatechin und 130 g Natriumhydroxid bei 95 bis 105 °C vorgelegt und bei dieser Temperatur unter Rühren 330 g Trifluorchlorethylen eingeleitet. Man destilliert anschließend den Ansatz bei 20 mbar über eine Kolonne und fängt eine Fraktion vom Siedepunkt 20 bis 60 °C/20 mbar in einer gut gekühlten Vorlage auf. Nachdem die wäßrige Phase abgetrennt wurde, verbleiben 332 g reines 2,2,3-Trifluor-1,4-benzodioxen (Ausbeute 87 % der Theorie) vom Siedepunkt 54-5 °C/16 mbar ; $n_D^{20} = 1,452\,5$.

Beispiel V 2

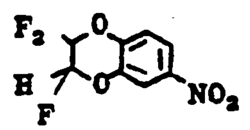

190 g 2,2,3-Trifluorbenzodioxen-(1,4) werden bei 5 °C vorgelegt ; bei dieser Temperatur wird eine Mischung aus 150 ml Salpetersäure (D 1,41) und 175 ml konz. Schwefelsäure zugetropft. Man rührt für 1 Stunde bei 10 °C, dann für 1 weitere Stunde bei 20 °C nach und erwärmt zum Schluß für 5 Minuten auf 40 °C. Der gekühlte Ansatz wird auf 500 g Eis gegossen und die organische Phase mit Methylenchlorid extrahiert. Nach Abdestillieren des Lösungsmittels wird der Rückstand im Vakuum destilliert. Man erhält 198 g 6-Nitro-2,2,3-trifluorbenzodioxen-(1,4) vom Siedepunkt 100-102 °C/1,33 mbar ; $n_D^{20} = 1,507\,8$.

Beispiel V 3

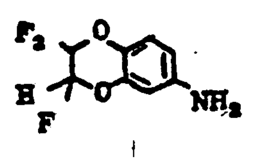

198 g 6-Nitro-2,2,3-trifluor-benzodioxen-(1,4) werden in 600 ml Ethanol gelöst und 20 g Raney-Nickel werden zugegeben. Unter Rühren wird Wasserstoff mit 50 bar aufgedrückt bis Sättigung bei 45 °C Innentemperatur erreicht ist. Nach Entspannen wird der Katalysator abfiltriert und das Filtrat destilliert. Man erhält 142 g 6-Amino-2,2,3-trifluor-benzodioxen-(1,4) vom Siedepunkt 125-127 °C/21 mbar, $n_D^{20} = 1,501$.

**Ansprüche**

1. Tetrachlorphthalamidsäuren der allgemeinen Formel

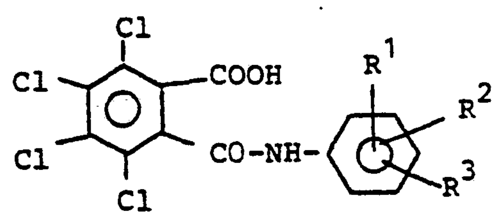

(I)

in welcher

$R^1$ für Trihalogenmethyl-, Trihalogenmethoxi- oder Trihalogenmethylmercapto-Gruppen steht,

$R^2$ für Wasserstoff, Halogen, Niederalkyl- oder Niederalkoxi-Gruppen steht, oder zusammen mit $R^1$ in o-Stellung $-O-CF_2-O-CF_2-$, $O-CF_2-O-$ und $-O-CF_2-CFX-O-$ bedeuten, wobei X für Wasserstoff, Chlor oder Fluor steht, und

$R^3$ für Wasserstoff, Halogen, Niederalkyl-, Niederalkoxi-, Niederalkylmercapto- oder Aroxi-Gruppen steht, wobei letztere gegebenenfalls durch Halogen, Methyl- und/oder Methoxygruppen substituiert sein können.

2. Verfahren zur Herstellung von Tetrachlorphthalamid säuren der Formel (I), dadurch gekennzeichnet, daß man Tetrachlorphthalsäureanhydrid der Formel

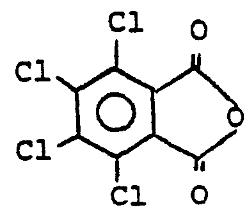

(II)

mit Aminen der Formel

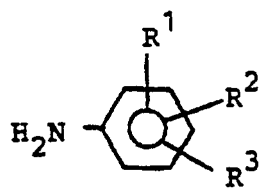

(III)

in welcher

$R^1$, $R^2$ und $R^3$ die in Anspruch 1 angegebene Bedeutung besitzen,

in Gegenwart eines Verdünnungsmittels umsetzt.

3. Bakterizide Pflanzenschutzmittel, gekennzeichnet durch einen Gehalt an mindestens einer Tetrachlorphthalamidsäure der Formel (I).

4. Verfahren zur Bekämpfung von pflanzenschädigenden Bakterien, dadurch gekennzeichnet, daß man Tetrachlorphthalamidsäuren der Formel (I) auf Bakterien oder ihren Lebensraum einwirken läßt.

5. Verwendung von Tetrachlorphthalamidsäuren der Formel (I) zur Bekämpfung von pfanzenschädigenden Bakterien.

6. Verfahren zur Herstellung von bakteriziden Pflanzenschutzmitteln, dadurch gekennzeichnet, daß man Tetrachlorphthalamidsäuren der Formel (I) mit Streckmitteln und/oder oberflächenaktiven Mitteln vermischt.

**Claims**

1. Tetrachlorophthalamic acids of the general formula

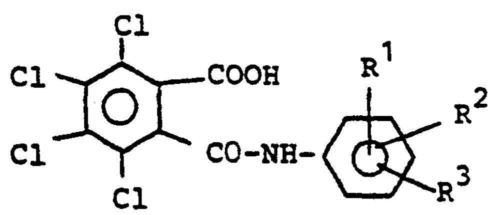

(I)

which

$R^1$ represents a trihalogenomethyl, trihalogenomethoxy or trihalogenomethylmercapto group,

$R^2$ represents hydrogen, halogen or a lower alkyl or lower alkoxy group, or, together with $R^1$ in the o-position, denotes $-O-CF_2-O-CF_2-$, $O-CF_2-O-$ or $-O-CF_2-CFX-O-$, wherein X represents hydrogen, chlorine or fluorine, and

$R^3$ represents hydrogen, halogen or a lower alkyl, lower alkoxy, lower alkylmercapto or aryloxy group, it being possible for the latter optionally to be substituted by halogen and/or methyl and/or methoxy groups.

2. Process for the preparation of tetrachlorophthalamic acids of the formula (I), characterised in that tetrachlorophthalic anhydride of the formula

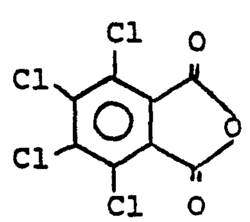

(II)

is reacted with amines of the formula

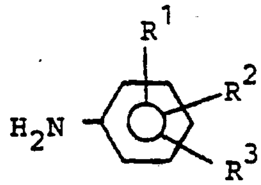

(III)

in which

$R^1$, $R^2$ and $R^3$ have the meaning given in Claim 1, in the presence of a diluent.

3. Bactericidal plant protection agents, characterised in that they contain at least one tetrachlorophthalamic acid of the formula (I).

4. Process for combating bacteria which are harmful to plants, characterised in that tetrachlorophthalamic acids of the formula (I) are allowed to act on bacteria or their environment.

5. Use of tetrachlorophthalamic acids of the formula (I) for combating bacteria which are harmful to plants.

6. Process for the preparation of bactericidal plant protection agents, characterised in that tetrachlorophthalamic acids of the formula (I) are mixed with extenders and/or surface-active agents.

**Revendications**

1. Acides tétrachlorophtalamiques de formule générale

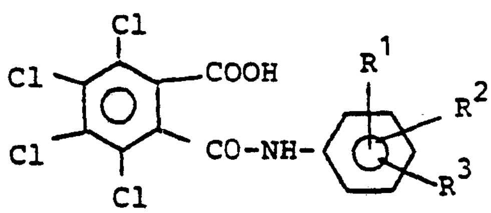

(I)

dans laquelle

$R^1$ représente des groupes trihalogénométhyle, trihalogénométhoxy ou trihalogénométhylmercapto,

$R^2$ représente l'hydrogène, des halogènes, des groupes alkyle inférieurs ou alkoxy inférieurs ou forment conjointement avec $R^1$ en position ortho un groupe $-O-CF_2-O-CF_2-$, $O-CF_2-O-$ et $-O-CF_2-CFX-O-$, où X est l'hydrogène, le chlore ou le fluor, et

$R^3$ représente l'hydrogène, un halogène, des groupes alkyle inférieurs, alkoxy inférieurs, alkylmercapto inférieurs ou aroxy, ces derniers pouvant éventuellement être substitués par un halogène, des groupes méthyle et/ou méthoxy.

2. Procédé de production d'acides tétrachlorophtalamiques de formule (I), caractérisé en ce qu'on fait réagir l'anhydride d'acide tétrachlorophtalique de formule

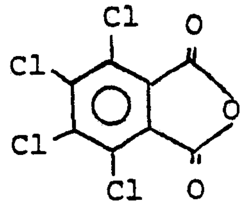

(II)

avec des amines de formule

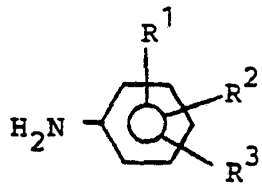

(III)

dans laquelle

$R^1$, $R^2$ et $R^3$ ont la définition indiquée dans la revendication 1, en présence d'un diluant.

3. Compositions bactéricides pour la protection des plantes, caractérisées par une teneur en au moins un acide tétrachlorophtalamique de formule (I).

4. Procédé de lutte contre des bactéries nuisibles aux plantes, caractérisé en ce qu'on fait agir des acides tétrachlorophtalamiques de formule (I) sur des bactéries ou sur leur milieu.

5. Utilisation d'acides tétrachlorophtalamiques de formule (I) dans la lutte contre des bactéries nuisibles aux plantes.

6. Procédé de préparation de compositions bactéricides pour la protection des plantes, caractérisé en ce qu'on mélange des acides tétrachlorophtalamiques de formule (I) avec des diluants et/ou des agents tensio-actifs.